# EUROPEAN PATENT SPECIFICATION

(11) **EP 3 377 137 B1**
(45) Date of publication and mention of the grant of the patent: **24.08.2022**
(21) Application number: 17757067.8
(22) Date of filing: 21.02.2017
(51) Int. Cl.: A61M 1/14, A61M 39/08, A61M 5/14, A61M 1/28, A61M 1/16

(54) **LINE HOLDER ASSEMBLY FOR DIALYSIS SYSTEM**
LEITUNGSHALTERANORDNUNG FÜR EIN DIALYSESYSTEM
ENSEMBLE PORTE-LIGNES POUR SYSTÈME DE DIALYSE

(30) Priority: 25.02.2016 US 201662299825 P
(43) Date of publication of application: 26.09.2018
(73) Proprietor: Fresenius Medical Care Holdings, Inc., Waltham, MA 02451-1457 (US)
(72) Inventor: BIEWER, John A., Waltham Massachusetts 02451 (US); WANDESFORDE, Alan, Lafayette California 94549 (US)
(74) Representative: Fish & Richardson P.C.
(86) International application number: PCT/US2017/018715
(87) International publication number: WO 2017/147071

(56) References cited:
- US-A- 5 876 371
- US-A1- 2005 077 436
- US-A1- 2006 237 597
- US-A1- 2006 237 597
- US-A1- 2007 149 914
- US-A1- 2012 123 322
- US-A1- 2014 061 421
- US-A1- 2015 014 249
- US-B1- 7 457 506
- US-B2- 7 160 087

## Description

### RELATED APPLICATIONS

This application claims priority to US Prov. App. 62/299,825 filed Feb. 25, 2016

### TECHNICAL FIELD

This application is related to the field of dialysis systems.

### BACKGROUND

Dialysis is a treatment used to support a patient with insufficient renal function. The two principal dialysis methods are hemodialysis and peritoneal dialysis.

During hemodialysis (HD), the patient's blood is passed through a dialyzer of a dialysis machine while also passing a dialysis solution or dialysate through the dialyzer. A semi-permeable membrane in the dialyzer separates the blood from the dialysate within the dialyzer and allows diffusion and osmosis exchanges to take place between the dialysate and the blood stream. These exchanges across the membrane result in the removal of waste products, including solutes like urea and creatinine, from the blood. These exchanges also regulate the levels of other substances, such as sodium and water, in the blood. In this way, the dialysis machine acts as an artificial kidney for cleansing the blood.

During peritoneal dialysis (PD), the patient's peritoneal cavity is periodically infused with dialysate. The membranous lining of the patient's peritoneum acts as a natural semi-permeable membrane that allows diffusion and osmosis exchanges to take place between the solution and the blood stream. These exchanges across the patient's peritoneum result in the removal of waste products, including solutes like urea and creatinine, from the blood, and regulate the levels of other substances, such as sodium and water, in the blood.

HD treatments are typically performed on a patient multiple times a week using an HD machine in a clinic or home environment with each treatment lasting a least a few hours. PD treatments are done several times a day on a patient, often at home and often performed overnight while a patient is asleep using an automated PD machine called a PD cycler. A PD cycler is designed to control the entire PD process so that it can be performed at home without clinical staff in attendance. Many PD cyclers are designed to automatically infuse, dwell, and drain dialysate to and from the patient's peritoneal cavity. The treatment typically lasts for several hours, often beginning with an initial drain cycle to empty the peritoneal cavity of used or spent dialysate. The sequence then proceeds through the succession of fill, dwell, and drain phases that follow one after the other. Each phase is called a cycle. Dialysis machines are typically equipped with interfaces for receiving inputs and providing information to users during treatments.

Dialysis machines may have a disposable set which has several connectors and often many feet of tubing (also referred to as "lines") used in connection with the dialysis treatment and through which medical fluid flows during the dialysis treatment. It is important that the connectors not touch dirty surfaces and become contaminated, which may lead to an infection such as peritonitis.

A line holder, that may be a plastic piece, may be used to hold the lines and connectors together to allow them to be anchored in an easy to reach area for the patient, which prevents them from separating and potentially falling to the ground becoming contaminated. The line holder may be positioned at the front of the dialysis machine to be within easy reach of the patient during set-up and easy to see for purposes of identifying the different connectors by colored clamps or caps. In many cases, room to mount the line holder on or near the dialysis machine is not readily available.

Accordingly, it would be desirable to provide an improved assembly for holding and organizing lines of a dialysis system.

US 2012/123322 describes a peritoneal dialysis system that includes a patient line state detector for detecting whether a patient line is primed before it is to be connected to the patient.

US 2005/077436 describes a device for organizing medical lines so as to facilitate identification and prevent entanglement and dislodgement of the lines that includes a rigid elongated base panel and an upwardly directed securing panel.

US 2006/237597 describes a tube holding apparatus that includes a mounting block having a block fastening end with a block fastening structure for removably securing the mounting block to a support surface and having a tube engaging end with several tube retaining structures. The block fastening structure includes a first fastener section of hook and loop fastener material secured to the block fastening end and a second fastener section of hook and loop material for mounting to a support surface to which the apparatus is to be secured, so that the mounting block can be removably secured to a support surface.

US 2007/149914 describes a package for use in a peritoneal dialysis treatment and a method for manufacturing of such a package. The package includes a line set, which comprises a first tubular line element, a second tubular line element and at least one component connected to the tubular line elements. The package comprises organizing means arranged to organize the line set such that no part of the line set extends across another part of the line set.

US 2015/014249 describes a method of controlling a medical fluid therapy machine display brightness.

### SUMMARY

According to an embodiment of the system described herein, a line holder assembly comprises an anchor base including a portion comprising a polymer material that, when in contact with a surface, resists movement of the line holder assembly across the surface. A nest is coupled to the anchor base in a position that is adjacent to an edge of a cart or table surface and oriented substantially perpendicular to a plane of the anchor base that is in contact with the cart or table surface. A line holder unit is secured to the nest and includes an opening to receive and secure a line of a medical device. The medical device may be a dialysis machine, such as a peritoneal dialysis machine. The polymer material portion may be an impact-resistant layer of the anchor base. The anchor base and the nest may be made of the polymer material. The polymer material may be silicone.

The nest may include at least one tab that secures the line holder unit in a position that is oriented substantially perpendicular to a plane of the anchor base that is in contact with the cart of table surface. The line holder unit may receive the line and hold the line in a position that is oriented substantially perpendicular to the plane of the anchor base.

A medical system may include a medical device having lines through which medical fluid flows during a medical treatment, and a line holder assembly.

### BRIEF DESCRIPTION OF THE DRAWINGS

Embodiments of the system described herein will be described in further detail below using the drawings, which are briefly described as follows.
FIG. 1 shows a schematic illustration of a dialysis system having lines held by a line holder assembly according to an embodiment of the system described herein.
FIGS. 2A-2C are schematic views of components of the line holder assembly according to an embodiment of the system described herein.
FIG. 3 is a picture of a dialysis system including a line holder assembly secured to a table surface and receiving lines from a dialysis machine (e.g., PD cycler) disposed on the table surface according to an embodiment of the system described herein.

### DETAILED DESCRIPTION

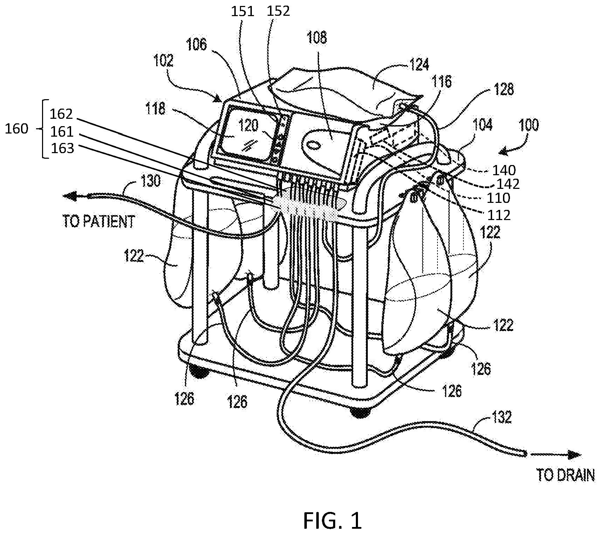

FIG. 1 shows a schematic illustration of a dialysis system having lines held by a line holder assembly according to an embodiment of the system described herein. The dialysis system may be a peritoneal dialysis (PD) system 100 that includes a PD machine or cycler 102 seated on a cart 104. Although a particular PD system is principally discussed herein, it is noted that the system described herein may be used in connection with various configurations and types of PD machines, including PD machines having cassettes, pumps (e.g., pneumatic based pumps and/or peristaltic pumps), gravity-driven cyclers, and/or with other types of dialysis systems and/or other medical devices having medical tubing or lines through which flows medical fluid. For example, the system described herein may also be used in connection with a home hemodialysis (HD) system.

The PD cycler 102 includes a housing 106, a door 108, and a cassette interface 110 that contacts a disposable PD cassette 112 when the cassette 112 is disposed within a cassette compartment formed between the cassette interface 110 and the closed door 108. A heater tray 116 is positioned on top of the housing 106. The heater tray 116 is sized and shaped to accommodate a bag of dialysate (e.g. a 5 liter bag of dialysate). The PD cycler 102 also includes a user interface such as a touch screen display 118 and additional control buttons 120 that can be operated by a user (e.g. a caregiver or a patient) to allow, for example, set up, initiation, and/or termination of a PD treatment.

As illustrated, dialysate bags 122 are suspended from support fingers on the sides of the cart 104, and a heater bag 124 is positioned in the heater tray 116. In other embodiments, the system described herein may be used in connection with dialysate supplied from bags positioned at other locations and/or via other mechanisms and devices for preparing and delivering dialysate to/from the dialysate machine and a patient. The dialysate bags 122 and the heater bag 124 are connected to the cassette 112 via dialysate bag lines 126 and a heater bag line 128, respectively. The dialysate bag lines 126 can be used to pass dialysate from dialysate bags 122 to the cassette 112 during use, and the heater bag line 128 can be used to pass dialysate back and forth between the cassette 112 and the heater bag 124 during use. In addition, a patient line 130 and a drain line 132 are connected to the cassette 112. The patient line 130 can be connected to a patient's abdomen via a catheter and can be used to pass dialysate back and forth between the cassette 112 and the patient's peritoneal cavity during use. The drain line 132 can be connected to a drain or drain receptacle and can be used to pass dialysate from the cassette 112 to the drain or drain receptacle during use. As further discussed elsewhere herein, one or more of the lines 126, 128, 130, 132 may be organized and/or secured using an embodiment of the system described herein.

The PD cycler 102 includes a control unit 140 (e.g. a processor) and one or more interface components or sensors 151, 152, such as a microphone/speaker assembly and/or a motion sensor that detects motion of a nearby user. The control unit 140 can receive signals from and transmit signals to the touch screen display 118, the control panel 120, the sensors 151, 152, and/or various other components of the PD system 100.

The PD cycler 102 may also include a data storage and/or transmission component 142 that may be coupled to the control unit 140 and that may enable the storage of data on the PD cycler and/or the transmission of data to and from the PD cycler 102. In various implementations, the data may include prescription information, treatment data and/or other therapy-based data and/or may include authorization information and/or other user profile-based information. In various implementations, the component 142 may include a wired connection to a network/Internet, an interface for receiving a physical storage unit, such as a universal serial bus (USB) memory unit, that may be used to transfer and receive data and/or wireless transmission components for transmitting or receiving data and/or other signals wirelessly. The wireless transmission components may include components for short range wireless transmission technologies, such as Bluetooth and/or near field communications (NFC) technologies, for communication with one or more peripheral devices and/or network transmission component. The communication may include transmitting and receiving data and/or other signals wirelessly via a telecommunications network and/or the Internet to/from one or more remote servers. In connection with transmission, data may be secured and/or encrypted via the control unit 140 using appropriate security and encryption protocols according to applicable laws and regulations governing transmission of sensitive data and/or protected medical information.

In an embodiment, a line holder assembly 160 according to the system described herein may include a molded nest 161 coupled to an anchor pad or base 162 having a flat bottom with sufficient surface area in contact with a table or other surface to secure the line holder assembly in place during use. The anchor base 162 is principally illustrated herein as having a semi-circular shape; however, other appropriate shapes may also be used in connection with the system described herein to perform the securing functionality of the line holder assembly 160 to the cart or table. The nest 161 is designed to extend around a cart or table edge and having tabs to accept a line holder unit 163 for holding and organizing the lines and which may be disposed in a vertical position. The nest 161 is shown coupled substantially perpendicular to the anchor base 162. In this way, the line holder assembly 160 may include components that contact multiple surfaces (e.g. front and top surfaces) of a cart or table on which the line holder assembly 160 to facilitate stability of the line holder assembly. The line holder assembly 160 may be beneficially used in connection with a home dialysis machine, such as the PD cycler 102, in which lines and connectors are desirably organized and anchored during a dialysis treatment. As discussed in detail herein, portions of the line holder assembly 160 may be made of one or more materials sufficiently structural to function to securely hold and protect lines in place while providing a frictional interface in contact with a surface to resist movement of the line holder assembly across the surface.

In an embodiment, the anchor base 162 may be made of and/or otherwise include a flexible, rubber-like material, such as silicone or other polymer or elastomer material, and thereby use the material's natural "stickiness" or frictional attachment to provide a fixation to the table or cart. A material such as silicone can attach to uneven surfaces, and since the entire anchor base 162 makes contact, it is more secure than multiple small suction cup feet. Further, silicone can be easily cleaned with soap and water, and cleaning restores the stickiness of the anchor base 162 if it has lost such stickiness after becoming dirty. In another embodiment, the anchor base 162 may be made of a different material and include a layer or pad made or silicone, or other polymer material, that is disposed on the underside of the anchor base 162 and contacts the cart or table surface.

The line holder assembly 160 may be conveniently repositioned which makes putting it away easy as well as making it portable for travel. The size of the line holder assembly may small, for example, smaller than the dialysis machine. Further, making the assembly small eliminates the need for right or left handed versions; there may be simply one version that can be placed on the right or left side of the dialysis machine without having to configure specifically the line holder assembly 160. An area of the bottom corner of the silicone pad of the anchor base 162 may be textured and/or otherwise made of a different material to make the area less sticky and provide an area which could be grabbed with fingers and used to help remove it from the table or cart surface when the patient wants to reposition it or travel with it.

One of the risks associated with line holders is if someone hits or bumps into the assembly, the force could break the line holder or cause pain to the patient. The system described herein with a flexible, rubber-like nest 161 and anchor base 162 is impact resistant in that it will have natural "give" which would lessen the force upon an impact, thereby reducing the probability of the line holder assembly 160 to move or break.

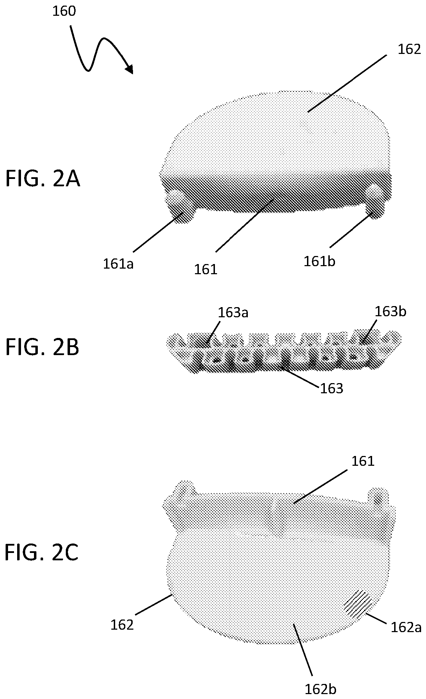

FIGS. 2A-2C are schematic views of components of the line holder assembly 160 according to an embodiment of the system described herein.

FIG. 2A is a view of the line holder assembly 160 showing the anchor base 162 that would contact a cart or table surface and the nest 161 onto which the line holder unit 163 may be inserted. Elements or tabs 161a, 161b are shown protruding out of the nest 162 and which may securely receive the line holder unit 163. The tabs 161a, 161b may position the line holder unit 163 in a vertical position being substantially perpendicular to the plane of the anchor base 162 when in contact with the cart or table surface.

FIG. 2B shows a schematic view of the line holder unit 163 that receives and secure lines of the dialysis machine. The line holder unit 163 includes openings 163a, 163b that may be disposed over the tabs 161a, 161b of the nest 161 to secure the line holder unit 163 in position.

FIG. 2C is a schematic view showing an underside of the line holder assembly 160, specifically the side of the anchor base 162 that would physically contact the cart or table surface, and the inside surface of the nest 161. The underside of the anchor base 162 may include a portion 162a that may be textured or otherwise include a different material that does not adhere to the table and thereby provide an area which could be grabbed with fingers and used to help remove it from the table or cart surface when the patient wants to reposition it or travel with it. In another embodiment, the anchor base 162 may be made of a different material, that is a material other than silicone, while including a layer, e.g. layer 162b, acting as a pad on the underside of the anchor base 162 that may be made of silicone or other polymer material as discussed herein and that is in physical contact with the cart or table surface.

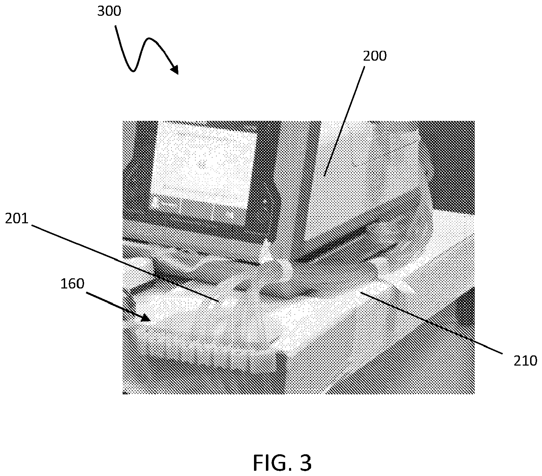

FIG. 3 is a picture of a dialysis system 300 including a line holder assembly 160 secured to a table surface 210 and receiving lines 201 from a dialysis machine (e.g., PD cycler) 200 disposed on the table surface 210 according to an embodiment of the system described herein. The line holder assembly 160 may be used during operation of the dialysis machine 200 when medical fluid is flowing in the lines 201 running to and from the dialysis machine 200 so as to so as to hold and organize lines. As further discussed elsewhere herein, the line holder assembly 160 may include portions made of an impact-resistant polymer material, such as silicone, that secures the line holder assembly 160 in place on the table surface 210 to resist movement of the line holder assembly 160 across the table surface and that is resistant to impacts. The nest 161 is coupled to the anchor base 162 in a position that is adjacent to an edge of the table surface 210 and oriented substantially perpendicular to a plane of the anchor base 162 that is in contact with the table surface 210. The system described herein may be advantageously used in connection with any type of dialysis machine, including home dialysis machines, that would benefit from the holding and organizing of lines running to and from the dialysis machine.

Various embodiments discussed herein may be combined with each other in appropriate combinations in connection with the system described herein. Additionally, in some instances, the order of steps in the flow diagrams, flowcharts and/or described flow processing may be modified, where appropriate. Additionally, although the system described herein has been discussed principally in connection with PD cyclers and treatment, in various embodiments the system described herein may also be used in connection with other medical machines and treatments using lines and connectors that are desirably organized and anchored.

## Claims

1. A line holder assembly (160) comprising:
an anchor base (162) including a portion comprising a polymer material that, when in contact with a surface (210), resists movement of the line holder assembly across the surface;
a nest (161) coupled to the anchor base in a position that is adjacent to an edge of a cart or table surface and oriented substantially perpendicular to a plane of the anchor base that is in contact with the cart or table surface; and
a line holder unit (163) that is secured to the nest and includes an opening to receive and secure a line of a medical device.

2. The line holder assembly according to claim 1, wherein the medical device is a dialysis machine.

3. The line holder assembly according to claim 2, wherein the dialysis machine is a peritoneal dialysis machine.

4. The line holder assembly according to claim 1, wherein the polymer material portion is an impact-resistant layer of the anchor base.

5. The line holder assembly according to claim 1, wherein the anchor base and the nest are made of the polymer material.

6. The line holder assembly according to claim 1, wherein the polymer material is silicone.

7. The line holder assembly according to claim 1, wherein the nest includes at least one tab (161a, 161b) that secures the line holder unit in a position that is oriented substantially perpendicular to a plane of the anchor base that is in contact with the cart of table surface.

8. The line holder assembly according to claim 7, wherein the line holder unit receives the line and holds the line in a position that is oriented substantially perpendicular to the plane of the anchor base.

9. A medical system (300) comprising:
a medical device having lines (201) through which medical fluid flows during a medical treatment; and
the line holder assembly according to any one of claims 1 to 8.

10. The medical system according to claim 9, wherein the medical device is a dialysis machine.

11. The medical system according to claim 10, wherein the dialysis machine is a peritoneal dialysis machine.

## Patentansprüche

1. Leitungshalteranordnung (160), umfassend:
eine Ankerbasis (162) mit einem Abschnitt, der ein Polymermaterial umfasst, das bei Kontakt mit einer Oberfläche (210) einer Bewegung der Leitungshalteranordnung über die Oberfläche widersteht;
ein Nest (161), das mit der Ankerbasis in einer Position verbunden ist, die sich neben einem Rand eines Wagens oder einer Tischoberfläche befindet und im Wesentlichen senkrecht zu einer Ebene der Ankerbasis, die mit dem Wagen oder der Tischoberfläche in Kontakt ist, orientiert ist; und
eine Leitungshaltereinheit (163), die am Nest befestigt ist und eine Öffnung zum Aufnehmen und Fixieren einer Leitung einer medizinischen Vorrichtung aufweist.

2. Leitungshalteranordnung nach Anspruch 1, wobei die medizinische Vorrichtung ein Dialysegerät ist.

3. Leitungshalteranordnung nach Anspruch 2, wobei das Dialysegerät ein Peritonealdialysegerät ist.

4. Leitungshalteranordnung nach Anspruch 1, wobei der Polymermaterialabschnitt eine schlagfeste Schicht der Ankerbasis ist.

5. Leitungshalteranordnung nach Anspruch 1, wobei die Ankerbasis und das Nest aus dem Polymermaterial bestehen.

6. Leitungshalteranordnung nach Anspruch 1, wobei das Polymermaterial Silikon ist.

7. Leitungshalteranordnung nach Anspruch 1, wobei das Nest mindestens eine Lasche (161a, 161b) aufweist, die die Leitungshaltereinheit in einer Position fixiert, die im Wesentlichen senkrecht zu einer Ebene der Ankerbasis, die mit dem Wagen oder der Tischoberfläche in Kontakt ist, orientiert ist.

8. Leitungshalteranordnung nach Anspruch 7, wobei die Leitungshaltereinheit die Leitung aufnimmt und die Leitung in einer Position festhält, die im Wesentlichen senkrecht zur Ebene der Ankerbasis orientiert ist.

9. Medizinisches System (300), umfassend:
eine medizinische Vorrichtung mit Leitungen (201), durch die medizinische Flüssigkeit während einer medizinischen Behandlung fließt; und
die Leitungshalteranordnung nach einem der Ansprüche 1 bis 8.

10. Medizinisches System nach Anspruch 9, wobei die medizinische Vorrichtung ein Dialysegerät ist.

11. Medizinisches System nach Anspruch 10, wobei das Dialysegerät ein Peritonealdialysegerät ist.

## Revendications

1. Ensemble porte-ligne (160) comprenant :
une base d'ancrage (162) comprenant une partie comprenant un matériau polymère qui, lors d'un contact avec une surface (210), résiste à un mouvement de l'ensemble porte-ligne en travers de la surface ;
un emboîtement (161) accouplé à la base d'ancrage dans une position qui est adjacente à un bord d'une surface d'un chariot ou d'une table et orientée sensiblement perpendiculairement à un plan de la base d'ancrage qui est en contact avec la surface du chariot ou de la table ; et
une unité de porte-ligne (163) qui est fixée à l'emboîtement et comprend une ouverture pour recevoir et assujettir une ligne d'un dispositif médical.

2. Ensemble porte-ligne selon la revendication 1, dans lequel le dispositif médical est une machine de dialyse.

3. Ensemble porte-ligne selon la revendication 2, dans lequel la machine de dialyse est une machine de dialyse péritonéale.

4. Ensemble porte-ligne selon la revendication 1, dans lequel la partie en matériau polymère est une couche résistante aux chocs de la base d'ancrage.

5. Ensemble porte-ligne selon la revendication 1, dans lequel la base d'ancrage et l'emboîtement sont réalisés en matériau polymère.

6. Ensemble porte-ligne selon la revendication 1, dans lequel le matériau polymère est de la silicone.

7. Ensemble porte-ligne selon la revendication 1, dans lequel l'emboîtement comprend au moins une languette (161a, 161b) qui assujettit l'unité de porte-ligne dans une position qui est orientée sensiblement perpendiculairement à un plan de la base d'ancrage qui est en contact avec la surface du chariot ou de la table.

8. Ensemble porte-ligne selon la revendication 7, dans lequel l'unité de porte-ligne reçoit la ligne et maintient la ligne dans une position qui est orientée sensiblement perpendiculairement au plan de la base d'ancrage.

9. Système médical (300) comprenant :
un dispositif médical comportant des lignes (201) à travers lesquelles s'écoule un fluide médical pendant un traitement médical ; et
l'ensemble porte-ligne selon l'une quelconque des revendications 1 à 8.

10. Système médical selon la revendication 9, dans lequel le dispositif médical est une machine de dialyse.

11. Système médical selon la revendication 10, dans lequel la machine de dialyse est une machine de dialyse péritonéale.
